(19) Europäisches Patentamt
European Patent Office
Office européen des brevets

(11) **EP 2 465 422 B1**

(12) **EUROPEAN PATENT SPECIFICATION**

(45) Date of publication and mention
of the grant of the patent:
**25.06.2014 Bulletin 2014/26**

(51) Int Cl.:
***A61B 5/021*** *(2006.01)*      ***A61B 5/02*** *(2006.01)*

(21) Application number: **11192296.9**

(22) Date of filing: **07.12.2011**

(54) **Circulatory function measurement device**

Kreislauffunktionsmessvorrichtung

Dispositif de mesure de la fonction circulatoire

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **16.12.2010 JP 2010280805
01.06.2011 JP 2011123830
16.11.2011 JP 2011251109**

(43) Date of publication of application:
**20.06.2012 Bulletin 2012/25**

(73) Proprietor: **Panasonic Corporation
Osaka 571-8501 (JP)**

(72) Inventors:
• **Ide, Kazuhiro
Chuo-ku
Osaka 540-6207 (JP)**
• **Kusakabe, Tomoya
Chuo-ku
Osaka 540-6207 (JP)**

(74) Representative: **Appelt, Christian W.
Boehmert & Boehmert
Pettenkoferstrasse 20-22
80336 München (DE)**

(56) References cited:
**JP-A- 2005 278 708      JP-A- 2007 044 364**

**Description**

BACKGROUND OF THE INVENTION

The present invention relates to a circulatory function measurement device that analyzes the condition of a living body based on pulse waves generated from the living body.

Early detection of arteriosclerosis has been enabled by measuring the circulatory arteries using a circulatory function measurement device. Japanese Laid-Open Patent Publication No. 2005-278708 describes an example of such a device. To measure a circulatory function using the device, a cuff is first placed around a subject's upper arm, and then the cuff is inflated with air. The pressure of the cuff (compressing pressure) presses the upper arm and generates pulse waves, which are used to measure the circulatory function, such as the degree of arteriosclerosis.

In the measurement using the device described in Japanese Laid-Open Patent Publication No. 2005-278708, the compressing pressure is slowly decreased at a constant velocity in a predetermined range of the compressing pressure, which includes the systolic pressure and diastolic pressure of a subject. The amplitude values of pulse waves are extracted from pressure signals, which are generated when the compressing pressure is decreased. The amplitude values of the pulse waves are then arranged in chronological order to obtain an envelope representing the amplitude values of the pulse waves. The compressing pressure corresponding to the maximum amplitude value of the pulse waves is then set as a boundary value. The boundary value divides the envelope into a high compressing pressure region, in which the compressing pressure is higher than the boundary value, and a low compressing pressure region, in which the compressing pressure is lower than the boundary value. The high and low compressing pressure regions each provide a characteristic value that can be used to determine the circulatory function (hardness of the blood vessels).

The characteristic value can be, for example, the difference between the boundary value and the compressing pressure corresponding to the amplitude value of a predetermined pulse wave in the high compressing pressure region or a difference between the boundary value and the compressing pressure corresponding to the amplitude value of a predetermined pulse wave in the low compressing pressure region. The characteristic values extracted from the different compressing pressure regions are compared with each other to determine the subject's degree of arteriosclerosis (circulatory function).

However, in the device described in Japanese Laid-Open Patent Publication No. 2005-278708, the characteristic values are obtained using only parts of changes in the amplitude values of the pulse waves. Thus, when a measurement is performed with the device for the second time under a different condition, the second measurement may yield a different result from the first measurement under different measurement conditions, such as the cuff being improperly set around the subject's upper arm. In such a case, the circulatory function measurement device cannot accurately measure the circulatory function.

JP 2007-044364 discloses a blood pressure plethysmography equipment which performs measurement of the vascular compliance and pulse wave velocity, wherein a main wave pulse extracting circuit extracts a pulse wave and accumulates the extracted pulse wave in the memory with the amplitude value of every pulse wave.

Summary of the invention

The present invention relates to a circulatory function measurement device according to claim 1. Claims 2 to 16 relate to particular advantages and realizations of the subject matter of claim 1.

One aspect of the present invention is a circulatory function measurement device including a compressing unit that compresses part of a subject's body. A compressing pressure detection unit detects a compressing pressure generated by the compressing unit. A compressing pressure control unit changes the compressing pressure. A pulse wave detection unit detects pulse wave information related to a pulse wave generated in the part of the body corresponding to the compressing pressure that is changed by the compressing pressure control unit. A pulse wave accumulated value calculation unit calculates a pulse wave accumulated value by accumulating plural pieces of the pulse wave information obtained during a period from when the compressing unit starts the compressing starts to when the compressing unit ends the compressing. A memory unit stores the pulse wave accumulated value in association with the compressing pressure. A circulatory function measurement unit measures a circulatory function using the pulse wave accumulated value.

BRIEF DESCRIPTION OF THE DRAWINGS

Fig. 1 is a block diagram of a blood vessel hardness measurement device according to a first embodiment of the present invention;

Fig. 2 is a graph showing the compressing pressure that changes from when the compressing of the upper arm artery starts to when the compressing ends;

Fig. 3 is a graph showing an envelope obtained from the amplitude values of the pulse waves and the compressing pressure from when the compressing starts to when the compressing ends;

Fig. 4 is a graph showing the relationship between the compressing pressure and the accumulated value of pulse waves;

Fig. 5 is a graph showing characteristic lines obtained from the pulse wave accumulated values and the compressing pressure;

Fig. 6 is a graph showing a characteristic line obtained by the pulse wave accumulate values and the compressing pressure in a second embodiment of the present invention;

Fig. 7 is a perspective view showing a simplified structure of a blood vessel;

Fig. 8(a) is a graph plotted using the gradients of characteristic lines obtained in different pressure ranges, and Fig. 8(b) is a chart showing the relationship between the compressing pressure and the blood vessel hardness;

Fig. 9(a) is a graph plotted using the gradients of characteristic lines obtained in different pressure ranges, and Fig. 9(b) is a chart showing the relationship between the compressing pressure and the blood vessel hardness;

Fig. 10(a) is a graph plotted using the gradients of characteristic lines obtained in different pressure ranges, and Fig. 10(b) is a chart showing the relationship between the compressing pressure and the blood vessel hardness;

Fig. 11(a) is a graph plotted using the gradients of characteristic lines obtained in different pressure ranges, and Fig. 11(b) is a chart showing the relationship between the compressing pressure and the blood vessel hardness;

Fig. 12(a) is a graph plotted using the gradients of characteristic lines obtained in different pressure ranges, and Fig. 12(b) is a chart showing the relationship between the compressing pressure and the blood vessel hardness;

Fig. 13(a) is a graph plotted using the gradients of characteristic lines obtained in different pressure ranges, and Fig. 13(b) is a chart showing the relationship between the compressing pressure and the blood vessel hardness;

Fig. 14(a) is a graph plotted using the gradients of characteristic lines obtained in different pressure ranges, and Fig. 14(b) is a chart showing the relationship between the compressing pressure and the blood vessel hardness;

Fig. 15(a) is a graph plotted using the gradients of characteristic lines obtained in different pressure ranges, and Fig. 15(b) is a chart showing the relationship between the compressing pressure and the blood vessel hardness;

Fig. 16 is a graph showing a characteristic line obtained from the pulse wave accumulated proportions and the compressing pressure in a third embodiment of the present invention;

Fig. 17(a) is a graph showing the relationship between the internal and external pressure difference of blood vessels, blood vessel volume, pulse pressure, and the pulse wave, and Fig. 17(b) is a graph showing an envelope obtained from the relationship between the pulse wave height and the internal and external pressure difference of the blood vessels;

Fig. 18(a) is a graph showing the relationship between the internal and external pressure difference of blood vessels, blood vessel volume, pulse pressure, and the pulse wave, and Fig. 18(b) shows an envelope defined by the relationship between the pulse wave height and the internal and external pressure difference of the blood vessels;

Fig. 19(a) is a graph showing a characteristic line obtained from the compressing pressure and the pulse wave accumulated proportions generated from soft blood vessels in a fourth embodiment of the present invention, and Fig. 19(b) is a graph showing a characteristic line obtained from the compressing pressure and the pulse wave accumulated proportions generated from hardened blood vessels in the fourth embodiment;

Fig. 20(a) is a graph showing a characteristic line obtained from the compressing pressure and the pulse wave accumulated proportions generated from soft blood vessels in a fifth embodiment of the present invention, and Fig. 20(b) shows a characteristic line obtained from the compressing pressure and the pulse wave accumulated proportions generated from hardened blood vessels in the fifth embodiment;

Fig. 21 is a graph showing an envelope obtained from the pulse wave amplitude values and the compressing pressure in a sixth embodiment of the present invention;

Fig. 22A is a graph showing a characteristic line obtained from the compressing pressure and the pulse wave accumulated proportions generated from soft blood vessels in a tenth embodiment of the present invention, and Fig. 22B shows a characteristic line obtained from the compressing pressure and the pulse wave accumulated proportions generated from hardened blood vessels in the tenth embodiment;

Fig. 23A is a graph showing a characteristic line obtained from the compressing pressure and the pulse wave accumulated proportions generated from soft blood vessels in an eleventh embodiment of the present invention, and Fig. 23B shows a characteristic line obtained from the compressing pressure and the pulse wave accumulated proportions generated from hardened blood vessels in the eleventh embodiment;

DETAILED DESCRIPTION OF THE PREFERRED EMBODIMENTS

First Embodiment

A blood vessel hardness measurement device according to a first embodiment of the present invention will now be described with reference to the drawings.

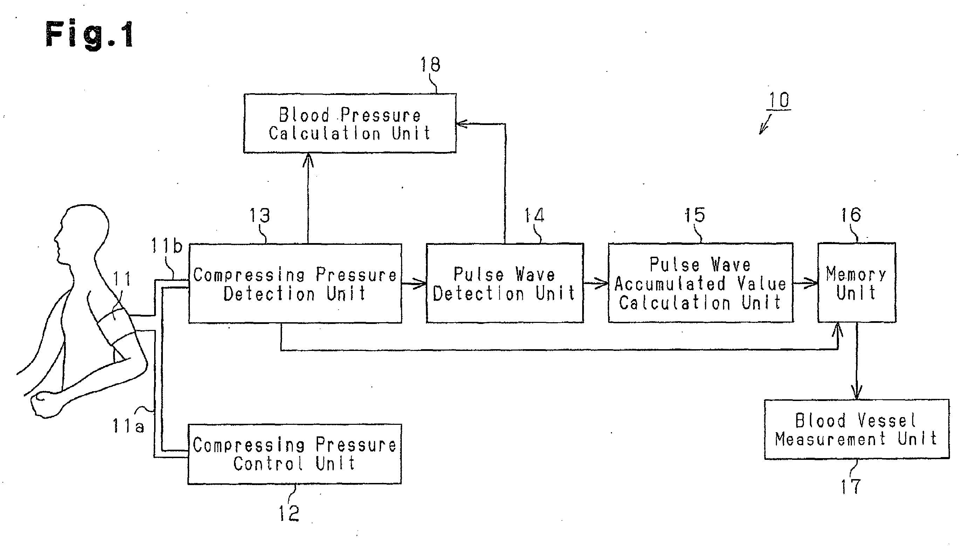

As shown in Fig. 1, a blood vessel hardness measurement device 10 includes a cuff 11, tubes 11a and 11b, a compressing pressure control unit 12, a compressing pressure detection unit 13, a pulse wave detection unit 14, a pulse wave accumulated value calculation unit 15, a memory unit 16, and a blood vessel hardness measurement unit 17. The cuff 11 functions as a compressing unit. The blood vessel hardness measurement unit 17 functions as a circulatory function measurement unit. The cuff 11 is made of rubber and is bag-shaped. The cuff 11 is set around a subject's upper arm, which is one part of the subject's body. The cuff 11 compresses the upper arm (the upper arm artery) of the subject in accordance with the pressure (the compressing pressure) of the cuff 11. The cuff 11 is connected to the compressing pressure control unit 12 via the tube 11a. The compressing pressure control unit 12 changes the compressing pressure of the cuff 11. The compressing pressure control unit 12 includes a pressurizing pump (not shown), which pressurizes the cuff 11, and a discharge valve (not shown), which depressurizes the cuff 11. The compressing pressure control unit 12 pressurizes and depressurizes the cuff 11 by driving and controlling the pressurizing pump and the discharge valve. In this manner, the compressing pressure control unit 12 changes the compressing pressure of the cuff 11.

The cuff 11 is also connected to the compressing pressure detection unit 13 via the tube 11b. The compressing pressure detection unit 13 detects the compressing pressure of the cuff 11. The compressing pressure detection unit 13 includes a pressure sensor and an A/D converter (neither shown). The compressing pressure detection unit 13 is provided with a pressure signal indicating the compressing pressure of the cuff 11 detected by the pressure sensor. The A/D converter of the compressing pressure detection unit 13 converts the pressure signal to a digital value indicating the compressing pressure (compressing pressure value). The compressing pressure detection unit 13 is electrically connected to the pulse wave detection unit 14 and provides the compressing pressure value to the pulse wave detection unit 14. The pulse wave detection unit 14 obtains pulse wave information, which is information indicating the value of a pulse wave generated in the upper arm, in accordance with the compressing pressure value, which is continuously changed by the compressing pressure control unit 12. The pulse wave detection unit 14 includes a filter circuit (not shown). The pulse wave detection unit 14 removes a predetermined frequency element, such as a direct current element, from the compressing pressure value provided from the compressing pressure detection unit 13 to generate a pulse wave signal. Then, the pulse wave detection unit 14 obtains the amplitude value of a pulse wave from the generated pulse wave signal.

The pulse wave accumulated value calculation unit 15, which is electrically connected to the pulse wave detection unit 14, calculates an accumulated value of pulse waves (a pulse wave accumulated value) by accumulating the amplitude values of pulse waves from when the compressing starts to when the compressing ends. More specifically, the pulse wave accumulated value calculation unit 15 calculates the pulse wave accumulated value by accumulating plural pieces of pulse wave information from when the compressing starts to when the compressing ends. The pulse wave accumulated value calculation unit 15 and the compressing pressure detection unit 13 are electrically connected to the memory unit 16. The memory unit 16 stores the compressing pressure values and the pulse wave accumulated values in association. More specifically, the memory unit 16 stores a pulse wave accumulated value in association with a predetermined compressing pressure value. The memory unit 16 continuously stores such associated compressing pressure values and pulse wave accumulated values from when the compressing starts to when the compressing ends.

The memory unit 16 is electrically connected to the blood vessel hardness measurement unit 17, which functions as the circulatory function measurement unit. The blood vessel hardness measurement unit 17 measures the hardness of the upper arm artery (hereafter referred to as the "blood vessel hardness"), which is an example of the circulatory function, based on a predetermined algorithm using the relationship between the compressing pressure corresponding to the detected amplitude value of the pulse wave and the pulse wave accumulated value. The blood vessel hardness measurement unit 17 includes a read-only memory (ROM), a random-access memory (RAM), and a central processing unit (CPU). The ROM may store programs for measuring the subject's blood vessel hardness and for driving and controlling each part of the blood vessel hardness measurement device 10. The RAM temporarily stores data generated during or after execution of the programs. The CPU reads the control programs and the like from the ROM and executes the read programs.

The compressing pressure detection unit 13 and the pulse wave detection unit 14 are electrically connected to the blood pressure calculation unit 18. The blood pressure calculation unit 18 calculates the subject's systolic pressure and diastolic pressure using a predetermined algorithm such as the oscillometric method. The blood pressure calculation unit 18 calculates the subject's systolic and diastolic pressures based on the relationship between the compressing pressure detected by the compressing pressure detection unit 13 and the amplitude value of the pulse wave detected by the pulse wave detection unit 14.

A method for measuring the blood vessel hardness using the blood vessel hardness measurement device 10 of the present embodiment will now be described.

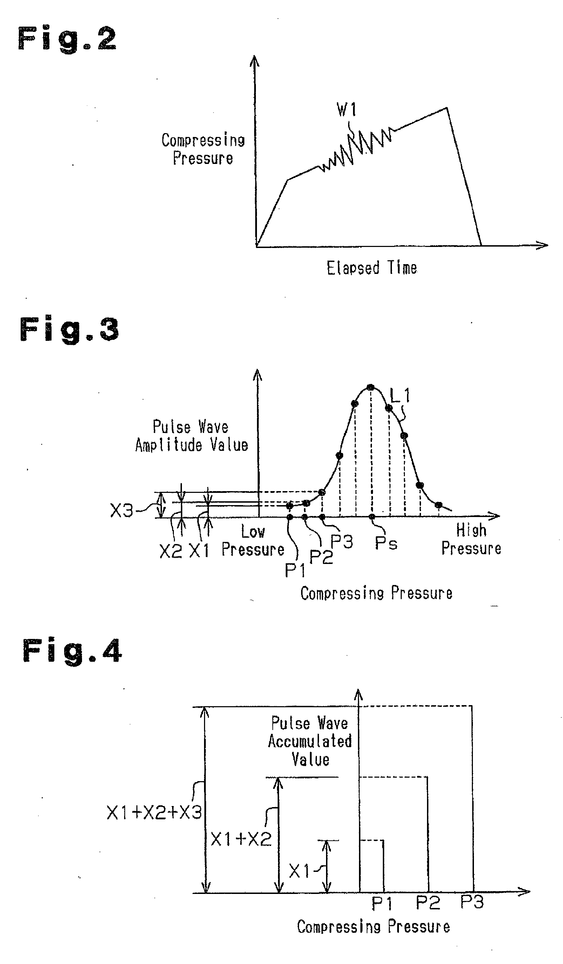

When the cuff 11, which is set around the subject's upper arm, is supplied with air from the pressurizing pump and inflated, the cuff 11 compresses the upper arm artery. Referring to Fig. 2, the compressing pressure control unit 12 then changes the compressing pressure of the cuff 11 so that the pressure gradually increases at a slow velocity from a pressure lower than the subject's expected diastolic pressure. When the compressing pressure reaches a predetermined pressure value, the compressing pressure control unit 12 decreases the compressing pressure by controlling the discharge valve. In this process of changing the compressing pressure of the cuff 11, a pulse wave W1 corresponding to the number of heartbeats is generated. Further, the pulse wave detection unit 14 detects the amplitude value of the pulse wave W1. The blood pressure calculation unit 18 calculates the systolic pressure and the diastolic pressure of the subject based on the relationship between the compressing pressure and the amplitude value of the pulse wave W1.

As shown by an envelope L1 in Fig. 3, the detected amplitude value of the pulse wave W1 changes as the compressing pressure of the cuff 11 changes. More specifically, as the compressing pressure of the cuff 11 increases from a low pressure toward higher pressures, the amplitude value of the pulse wave W1 initially increases in small amounts and then gradually increases in larger amounts until reaching its maximum at a predetermined compressing pressure Ps. From the maximum value, the amplitude value of the pulse wave W1 decreases gradually. The memory unit 16 stores the amplitude value of the pulse wave W1 in a manner that each amplitude value is associated with the corresponding compressing pressure.

In one example, the pulse wave detection unit 14 detects an amplitude value X1 of the pulse wave W1 at a compressing pressure P1, an amplitude value X2 of the pulse wave W1 at a compressing pressure P2, and an amplitude value X3 of the pulse wave W1 at a compressing pressure P3. In this case, as shown in Fig. 4, the pulse wave accumulated value calculation unit 15 calculates a pulse wave accumulated value X1 at the compressing pressure P1, a pulse wave accumulated value X1+X2 at the compressing pressure P2, and a pulse wave accumulated value X1+X2+X3 at the compressing pressure P3. In this manner, the pulse wave accumulated value calculation unit 15 accumulates the amplitude values of the pulse wave W1 in chronological order as the compressing pressure changes. The pulse wave accumulated value calculation unit 15 accumulates the amplitude values of the pulse wave W1 when the compressing of the upper arm artery starts to when the compressing ends. The memory unit 16 stores the pulse wave accumulated values in association with the corresponding compressing pressures.

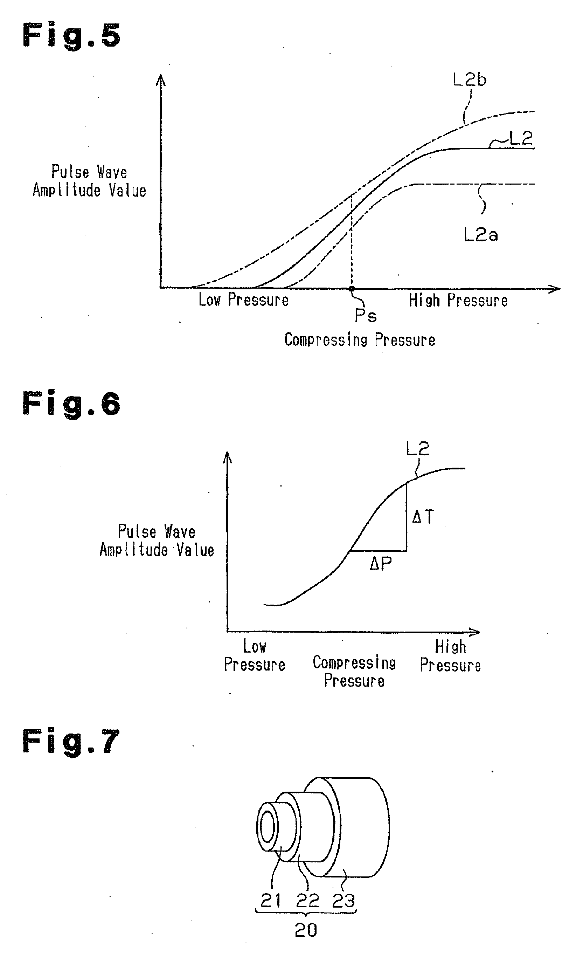

As shown by a characteristic line L2 in Fig. 5, the calculated pulse wave accumulated value increases as the compressing pressure of the cuff 11 changes. More specifically, as the compressing pressure of the cuff 11 increases from a low pressure toward higher pressures, the pulse wave accumulated value initially increases in small amounts and suddenly increases at the predetermined compressing pressure Ps. Subsequently, as the compressing pressure further increases from the predetermined compressing pressure Ps, the pulse wave accumulated value increases in amounts that gradually become smaller. The predetermined compressing pressure Ps is the compressing pressure value corresponding to the pulse wave accumulated value that was increased the most. The blood vessel hardness measurement unit 17 measures the blood vessel hardness using a predetermined algorithm based on the relationship between the pulse wave accumulated value and the compressing pressure stored in the memory unit 16.

In Fig. 5, a single-dashed line L2a indicates a characteristic line obtained for soft blood vessels, and a double-dashed line L2b indicates a characteristic line obtained for hardened blood vessels. The pulse wave accumulated values for the soft blood vessel are smaller than the pulse wave accumulated values for the hard blood vessel. Each pulse wave accumulated value corresponds to the amount of work required to squeeze the blood vessel. When the blood vessel is soft, a small amount of work corresponding to the compressing pressure is required to squeeze the vessel. In contrast, when the blood vessel is hard, a large amount of work corresponding to the compressing pressure is required to squeeze the blood vessel.

The present embodiment has the advantages described below.

(1) The pulse wave accumulated value calculation unit 15 calculates the pulse wave accumulated value by accumulating the amplitude values of the pulse waves W1 in chorological order as the compressing pressure changes. The blood vessel hardness measurement unit 17 measures the blood vessel hardness based on the relationship between the calculated pulse wave accumulated value and the compressing pressure. This measures the subject's blood vessel hardness more accurately than when using parts of changes in the amplitude values of the pulse waves W1 to do so.

(2) The blood vessel hardness measurement unit 17 measures the blood vessel hardness using the characteristic line L2 obtained from the relationship between the pulse wave accumulated value and the compressing pressure. The pulse wave accumulated value corresponds to the amount of work required to squeeze the blood vessel. The pulse wave accumulated values for hardened blood vessels are larger than those for soft blood vessels. The use of the pulse wave accumulated value and the compressing pressure allows for accurate determination of the blood vessel hardness.

(3) The blood vessel hardness measurement device 10 measures the subject's blood vessel hardness using the pulse wave W1 generated when measuring the subjects blood pressure measurement with the upper arm. Thus, the subject's blood vessel hardness can be calculated when normal blood pressure measurement is completed. This shortens the measurement time as compared when the blood pressure measurement and blood vessel hardness measurement are separately performed. Thus, the convenience of the blood vessel hardness measurement device 10 is increased.

Second Embodiment

A blood vessel hardness measurement device according to a second embodiment of the present invention will now be described with reference to Figs. 6 to 15B. To avoid redundancy, like or same reference numerals are given to those components that are the same as the corresponding components of the first embodiment. Such components will not be described in detail.

Referring to Fig. 6, the blood vessel hardness measurement unit 17 measures the blood vessel hardness based on the amount by which the pulse wave accumulated value changes as the compressing pressure changes, that is, the rate of change of the characteristic line L2. The blood vessel hardness measurement unit 17 calculates the rate of change $\Delta T/\Delta P$ (the gradient of the characteristic line L2), which is the rate of change of the pulse wave accumulated values corresponding to the compressing pressures stored in the memory unit 16. The blood vessel hardness measurement unit 17 measures the blood vessel hardness based on the gradient of the characteristic line L2.

The characteristic line L2 indicating the blood vessel hardness has different gradients for different compressing pressures. As shown in Fig. 7, a blood vessel wall 20 includes an inner layer 21, a middle layer 22, and an outer layer 23. The inner layer 21, the middle layer 22, and the outer layer 23 are each formed from elastin and collagen. The inner layer 21, the middle layer 22, and the outer layer 23 contain elastin and collagen at different ratios. Due to the different composition ratios, the layers have different dynamic properties at different compressing pressures. As a result, the characteristic line L2 exhibit different gradients under different compressing pressures. Elastin has a low rigidity, whereas collagen has a high rigidity. A normal blood vessel has a high content of elastin in the middle layer 22, and a high content of collagen in the outer layer 23. In the low and intermediate compressing pressure regions, the middle layer 22 mainly contributes to the flexibility of the blood vessel. In the high compressing pressure region, the outer layer 23 mainly contributes to flexibility of the blood vessel. The dynamic properties differ between the inner side and outer side of the middle layer 22. Thus, the measurement of blood vessel hardness at multiple measurement points allows the hardened portion of the blood vessel to be located. Specifically, such measurement allows for a determination of which of the middle layer 22 and the outer layer 23 of the blood vessel is hardened.

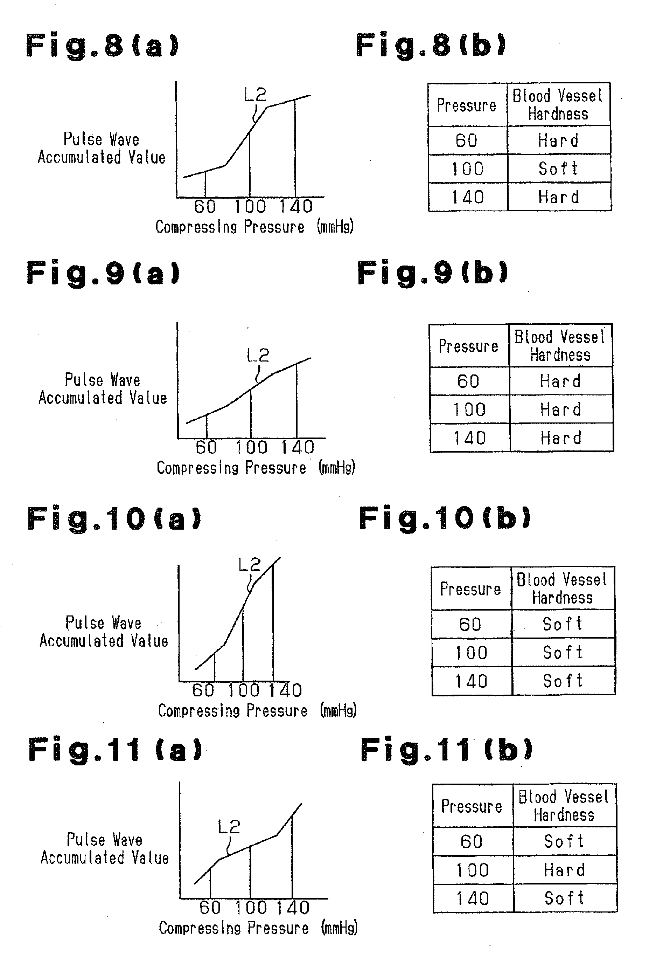

Figs. 8A to 15A are eight graphs showing straight lines plotting gradients of changes in the pulse wave accumulated values at the compressing pressure ranges of $60\pm20$ mmHg, $100\pm20$ mmHg, and $140\pm20$ mmHg. When no calculated pulse wave accumulated values correspond to the compressing pressure ranges of $60\pm20$ mmHg, $100\pm20$ mmHg, and $1440\pm20$ mmHg, the values corresponding to those ranges may be interpolated through, for example, linear interpolation, using values that are close to the compressing pressures.

In the graph shown in Fig. 8(a), the characteristic line L2 has a small gradient in the pressure range including the reference compressing pressure of 60 mmHg, a large gradient in the pressure range including the reference compressing pressure of 100 mmHg, and a small gradient in the pressure range including the reference compressing pressure of 140 mmHg. As shown in Fig. 8(b), blood vessel hardness is thus "hard" in the pressure range including the reference compressing pressure of 60 mmHg, "soft" in the pressure range including the reference compressing pressure of 100 mmHg, and "hard" in the pressure range including the reference compressing pressure of 140 mmHg. In this case, the blood vessels are assumed to be hard at the inner side of the middle layer 22, normal at the outer side of the middle layer 22, and hard at the outer layer 23.

In the graph shown in Fig. 9(a), the characteristic line L2 has a small gradient in the pressure ranges including the reference compressing pressures of 60, 100, and 140 mmHg. As shown in Fig. 9(b), the blood vessel hardness is thus "hard" in the pressure ranges including the reference compressing pressures of 60,100, and 140 mmHg. In this case, the blood vessels are hard at the inner and outer sides of the middle layer 22, and also hard at the outer layer 23.

In the graph shown in Fig. 10(a), the characteristic line L2 has a large gradient in the pressure ranges including the reference compressing pressures of 60, 100, and 140 mmHg. As shown in Fig. 10(b), the blood vessel hardness is thus "soft" in the pressure ranges including the reference compressing pressures of 60, 100, and 140 mmHg. In this case, the blood vessel hardness is normal at the inner and outer sides of the middle layer 22, and also normal at the outer layer 23.

In the graph shown in Fig. 11 (a), the characteristic line L2 has a large gradient in the pressure range including the reference compressing pressure of 60 mmHg, a small gradient in the pressure range including the reference compressing pressure of 100 mmHg, and a large gradient in the pressure range including the reference compressing pressure of 140 mmHg. As shown in Fig. 11 (b), the blood vessel hardness is thus "soft" in the pressure range including the reference compressing pressure of 60 mmHg, "hard" in the pressure range including the reference compressing pressure of 100 mmHg, and "soft" in the pressure range including the reference compressing pressure of 140 mmHg. In this case, the blood vessels are normal at the inner side of the middle layer 22, hard at the outer side of the middle layer 22, and normal at the outer layer 23.

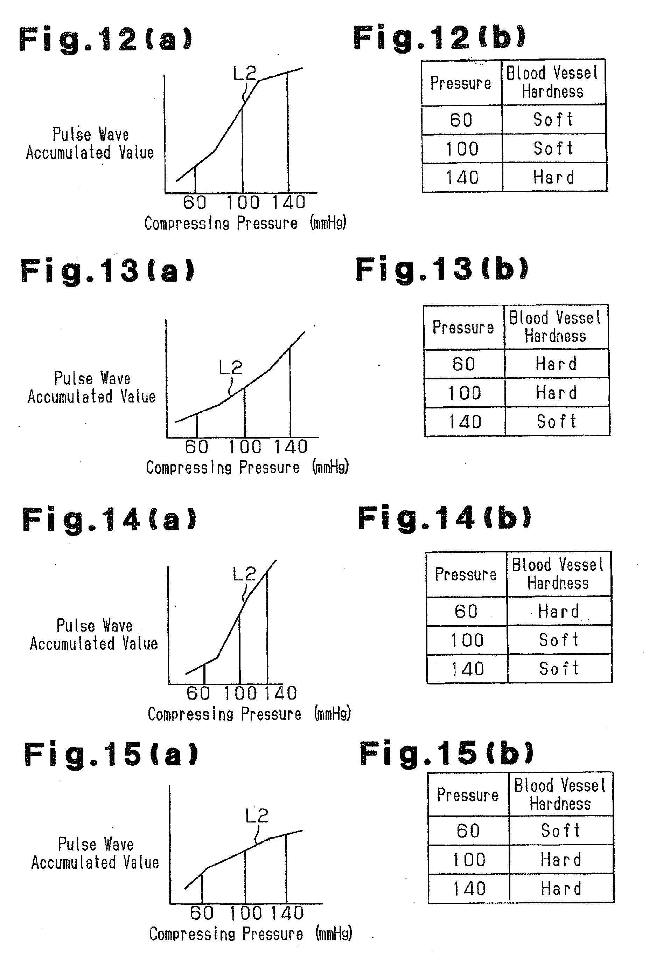

In the graph shown in Fig. 12(a), the characteristic line L2 has a large gradient in the pressure ranges including the reference compressing pressures of 60 and 100 mmHg, and a small gradient in the pressure range including the reference compressing pressure of 140 mmHg. As shown in Fig. 12(b), the blood vessel hardness is thus "soft" in the pressure ranges including the reference compressing pressures of 60 and 100 mmHg, and "hard" in the pressure range including the reference compressing pressure of 140 mmHg. In this case, the blood vessels are normal at the inner and outer sides of the middle layer 22, and hard at the outer layer 23.

In the graph shown in Fig. 13(a), the characteristic line L2 has a small gradient in the pressure ranges including the reference compressing pressures of 60 and 100 mmHg, and a large gradient in the pressure range including the reference compressing pressure of 140 mmHg. As shown in Fig. 13(b), the blood vessel hardness is thus "hard" in the pressure ranges including the reference compressing pressures of 60 and 100 mmHg, and "soft" in the pressure range including the reference compressing pressure of 140 mmHg. In this case, the blood vessels are hard at the inner and outer sides of the middle layer 22, and normal at the outer layer 23.

In the graph shown in Fig. 14(a), the characteristic line L2 has a small gradient in the pressure range including the reference compressing pressure of 60 mmHg, and a large gradient in the pressure ranges including the reference compressing pressures of 100 and 140 mmHg. As shown in Fig. 14(b), the blood vessel hardness is thus "hard" in the pressure range including the reference compressing pressure of 60 mmHg, and "soft" in the pressure ranges including the reference compressing pressures of 100 and 140 mmHg. In this case, the blood vessels are hard at the inner side of the middle layer 22, normal at the outer side of the middle layer 22, and normal at the outer layer 23.

In the graph shown in Fig. 15(a), the characteristic line L2 has a large gradient in the pressure range including the reference compressing pressure of 60 mmHg, and a small gradient in the pressure ranges including the reference compressing pressures of 100 and 140 mmHg. As shown in Fig. 15(b), the blood vessel hardness is thus "soft" in the pressure range including the reference compressing pressure of 60 mmHg, and "hard" in the pressure ranges including the reference compressing pressures of 100 and 140 mmHg. In this case, the blood vessels are normal at the inner side of the middle layer 22, hard at the outer side of the middle layer 22, and hard at the outer layer 23.

The hardness of the entire blood vessel can be expressed by the equation shown below:

Equation 1
$$\text{Blood Vessel Hardness} = A \cdot (\text{Blood Vessel Hardness at 60 mmHg}) + B \cdot (\text{Blood Vessel Hardness at 100 mmHg}) + C \cdot (\text{Blood Vessel Hardness at 140 mmHg})$$

In the equation, A, B, and C are weighting coefficients. The equation 1 may be used to obtain the blood vessel hardness of the entire blood vessel.

In addition to advantages (1) to (3) of the first embodiment, the second embodiment has the advantages described below.

(4) The blood vessel hardness measurement unit 17 measures the blood vessel hardness based on the rate of change in the characteristic line L2. More specifically, the blood vessel hardness measurement unit 17 measures the blood vessel hardness using parts of changes in the characteristic line L2 obtained from the pulse wave accumulated values, which are generated by accumulating the amplitude values of the pulse waves W1, and the compressing pressure.

The blood vessel hardness measurement unit 17 thus allows for further accurate measurement of the blood vessel hardness.

(5) The characteristic line L2 representing the blood vessel hardness has different gradients for different compressing pressures. The device of the present embodiment measures the blood vessel hardness in a plurality of pressure ranges using the rate of change $\Delta T/\Delta P$ of the pulse wave accumulated values. Thus, the measurement of the blood vessel hardness at multiple measurement points allows the hardened portion of the blood vessel to be located. Specifically, such measurement allows for a determination as to which of the middle layer 22 and the outer layer 23 of the blood vessels is hardened. Further, the hardness of all the blood vessels can be determined.

Third Embodiment

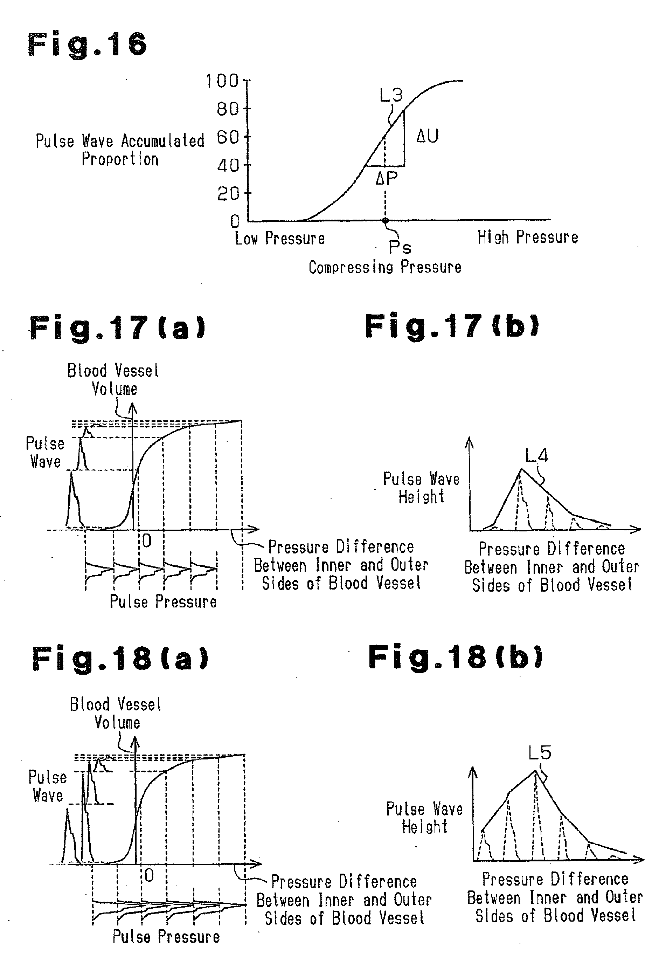

A blood vessel hardness measurement device according to a third embodiment of the present invention will now be described with reference to Fig. 16. In the present embodiment, the pulse wave accumulated value calculation unit 15 uses, as a reference value, the largest one of the pulse wave accumulated values obtained from when the accumulating of the amplitude values of the pulse waves W1 starts to when the accumulating ends, and uses the proportion of each pulse wave accumulated value relative to the largest value (hereafter referred to as the "pulse wave accumulated proportion") to determine the blood vessel hardness. The memory unit 16 stores the pulse wave accumulated proportions and the compressing pressures in association with each other.

The blood vessel hardness measurement unit 17 measures the blood vessel hardness based on a characteristic line L3 shown in Fig. 16. The blood vessel hardness measurement unit 17 calculates the rate of change $\Delta U/\Delta P$ (the gradient of the characteristic line L3), which is the rate of change of the pulse wave accumulated proportions corresponding to the compressing pressures stored in the memory unit 16. The blood vessel hardness measurement unit 17 measures the blood vessel hardness based on the gradients of the characteristic line L3. The blood vessel hardness measurement unit 17 measures the blood vessel hardness using, for example, a map associating the gradient of the characteristic line L3 and the blood vessel hardness.

When a blood vessel is compressed, pressure from the blood (internal pressure) and external pressure (compressing pressure) are applied to a blood vessel wall 20. When the compressing pressure is substantially the same as an average blood pressure, the difference between the internal and external pressures applied to the blood vessel wall 20 is substantially zero. Thus, no load is applied to the blood vessel wall 20. In this state, the compliance of the blood vessel (compliance of the blood vessel wall with respect to pulsation) is maximum, and the amount of change in the volume of the blood vessels is maximum under a fixed pulse pressure. In this state, the amplitude value of the pulse wave W1 is maximum.

When the pulse pressure is small as shown in Fig. 17(a), the relationship between the pulse wave height and the difference in internal and external pressures of the blood vessel is represented by an envelope L4 shown in Fig. 17(b). When the pulse pressure is large as shown in Fig. 18(a), the relationship between the pulse wave height and the internal and external pressure difference of the blood vessel is represented by an envelope L5 shown in Fig. 18(b). The envelope L4 shown in Fig. 17(b) and the envelope L5 shown in Fig, 18(b) differ from each other in gradient as well as in the pressure width. Under different pulse pressures, the envelopes obtained for the same blood vessel hardness differ from each other. Thus, the blood vessel hardness cannot be accurately measured. To solve this problem, the device of the present embodiment measures the blood vessel hardness with the pulse wave accumulated proportions. Thus, the blood vessel hardness can be accurately measured without being affected by the degree of a pulse pressure.

The third embodiment has the advantages described below.

(6) The blood vessel hardness measurement unit 17 measures the blood vessel hardness using the pulse wave accumulated proportions. Thus, the blood vessel hardness can be accurately measured without being affected by the degree of a pulse pressure.

Fourth Embodiment

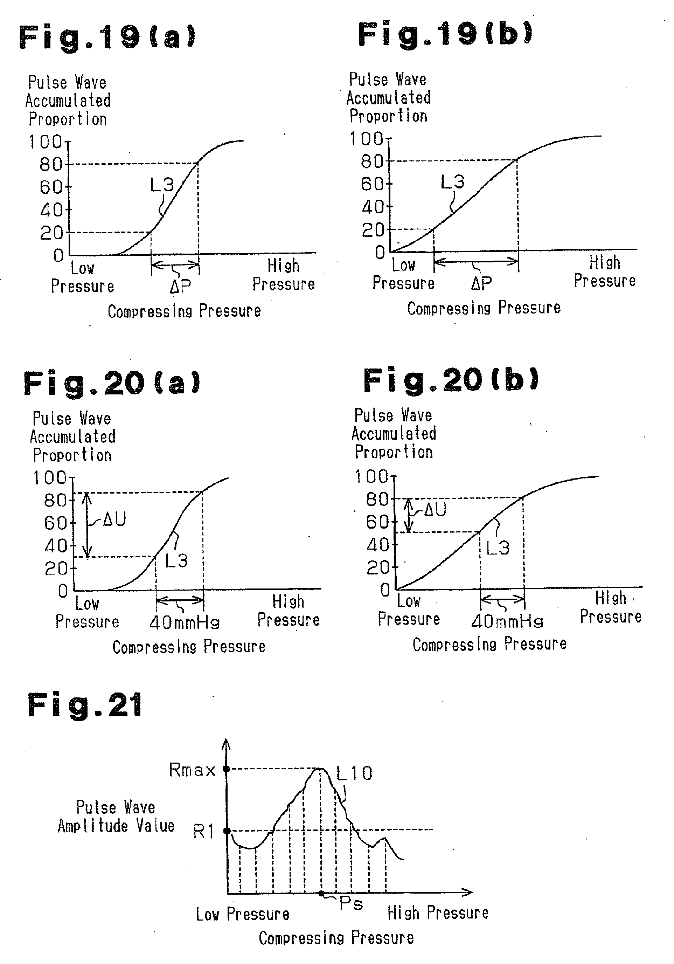

A blood vessel hardness measurement device according to a fourth embodiment of the present invention will now be described with reference to Figs. 19(a) and 19(b). In the present embodiment, the compressing pressure detection unit 13 detects a change $\Delta P$ of the compressing pressure that occurs when the pulse wave accumulated proportion changes from 20% to 80%. A blood vessel hardness measurement unit 17 measures the blood vessel hardness based on the detected change $\Delta P$ of the compressing pressure.

Fig. 19(a) is a graph showing a characteristic line L3 obtained for soft blood vessels. Fig. 19(b) is a graph showing a characteristic line L3 obtained for hardened blood vessels. As apparent from the comparison between the graphs of Figs. 19(a) and 19(b), the change $\Delta P$ of the compressing pressure that occurs in the pulse wave accumulated proportion range of 20% to 80% is smaller for the soft blood vessels than for the hard blood vessels. The blood vessel hardness measurement unit 17 then measures the blood vessel hardness using, for example, a map associating the compressing pressure change $\Delta P$ and the blood vessel hardness.

The fourth embodiment has the advantage described below.

(7) In the present embodiment, the blood vessel hardness is measured simply using the change $\Delta P$ of the compressing pressure. This reduces the load on the blood vessel hardness measurement unit 17.

Fifth Embodiment

A blood vessel hardness measurement device according to a fifth embodiment of the present invention will now be described with reference to Figs. 20(a) and 20(b). In the present embodiment, the blood vessel hardness measurement unit 17 measures the blood vessel measurement based on an change $\Delta U$ in the pulse wave accumulated proportion occurring when the compressing pressure changes in a range including a reference compressing pressure of 40 mmHg.

Fig. 20(a) is a graph showing a characteristic line L3 obtained for soft blood vessels. Fig. 20(b) is a graph showing a characteristic line L3 obtained for hardened blood vessels. As apparent from the comparison between the graphs of Figs. 20(a) and 20(b), the change $\Delta U$ in the pulse wave accumulated proportion that occurs in the compressing pressure range including the reference compressing pressure of 40 mmHg is smaller for the soft blood vessels than for the hard blood vessels. The blood vessel hardness measurement unit 17 measures the blood vessel hardness using, for example, a map associating the pulse wave accumulated proportion change $\Delta U$ and the blood vessel hardness.

The fifth embodiment has the advantages described below.

(8) In the present embodiment, the blood vessel hardness is measured simply using the change $\Delta U$ of the pulse wave accumulated proportion. This reduces the load on the blood vessel hardness measurement unit 17.

Sixth Embodiment

A blood vessel hardness measurement device according to a sixth embodiment of the present invention will now be described with reference to Fig. 21. In the present embodiment, a pulse wave accumulated value calculation unit 15 calculates a pulse wave accumulated value by accumulating the amplitude values of pulse waves W1 from which the amplitude values of abnormal pulse waves W1 detected by a pulse wave detection unit 14 are removed.

Generally, the amplitude value of the pulse wave W1 is first increased by a small amount and then gradually increased by a larger amount as the compressing pressure of the cuff 11 changes from a low pressure toward higher pressures. The amplitude value of the pulse wave W1 then becomes maximum at a predetermined compressing pressure Ps. After reaching the maximum value, the amplitude value of the pulse wave W1 gradually decreases. However, the amplitude value may be locally decrease or increase due to, for example, movement of the body during the measurement. The amplitude value of an abnormal pulse wave W1 detected by the pulse wave detection unit 14 affects the pulse wave accumulated value calculated by the pulse wave accumulated value calculation unit 15.

In Fig. 21, Rmax is the maximum amplitude value of the pulse wave W1 at the predetermined compressing pressure Ps. In the range in which the amplitude value of the pulse wave W1 is R1 to Rmax, the pulse wave accumulated value calculation unit 15 determines that the amplitude value of the pulse wave W1 detected by the pulse wave detection unit 14 is normal. In a range in which the amplitude value of the pulse wave W1 is less than R1, the pulse wave accumulated value calculation unit 15 determines that the amplitude value of the pulse wave W1 detected by the pulse wave detection unit 14 is abnormal. The pulse wave accumulated value calculation unit 15 in the sixth embodiment only accumulates the amplitude values of the pulse waves W1 exceeding the value R1. The pulse wave accumulated value calculation unit 15 does not accumulate any amplitude values of the pulse waves W1 that are less than the value R1.

The sixth embodiment has the advantages described below.

(9) The pulse wave accumulated value calculation unit 15 calculates the pulse wave accumulated value by accumulating only the amplitude values of normal pulse waves W1 measured by the pulse wave detection unit 14 and not accumulating the amplitude values of abnormal pulse waves W1. This prevents abnormal values from affecting the pulse wave accumulated value that is used to measure the blood vessel hardness. Thus, the blood vessel hardness can be accurately measured:

Seventh Embodiment

A blood vessel hardness measurement device according to a seventh embodiment of the present invention will now be described with reference to Figs. 19(a) and 19(b).

As described above, the pulse wave accumulated value corresponds to the amount of work required to squeeze the blood vessels. The amount of work required to squeeze the blood vessels changes depending on the subject's blood vessel hardness and systolic pressure BH. Under the same blood vessel hardness, the amount of work required to change the pulse wave accumulated proportion is larger when the subject's systolic pressure BH is higher. Thus, under the same blood vessel hardness, a change $\Delta P$ of the compressing pressure occurring when the pulse wave accumulated proportion changes by a predetermined amount has a tendency of becoming larger as the subject's systolic pressure BH becomes higher.

In the present embodiment, the compressing pressure detection unit 13 detects the change $\Delta P$ of the compressing pressure corresponding to the pulse wave accumulated proportion range of 20 to 80%. The blood vessel hardness measurement unit 17 divides the detected change $\Delta P$ of the compressing pressure by the systolic pressure BH of the subject. More specifically, the blood vessel hardness measurement unit 17 corrects the compressing pressure change $\Delta P$ and obtains a corrected value $\Delta P/BH$. The blood vessel hardness measurement unit 17 then measures the blood vessel hardness using, for example, a map associating the corrected value $\Delta P/BH$ and the blood vessel hardness.

In addition to advantages (1), (3), (6), and (7), which are mentioned above, the seventh embodiment has the advantage described below.

(10) The blood vessel hardness measurement unit 17 corrects the compressing pressure change $\Delta$P using circulatory information of the subject that can be obtained using the pulse wave accumulated values. More specifically, the blood vessel hardness measurement unit 17 corrects the pressure change value by dividing the compressing pressure change $\Delta$P by the systolic pressure BH, which is the subject's biological information. The blood vessel hardness measurement unit 17 then determines the subject's blood vessel hardness using the corrected value $\Delta$P/BH. Thus, the blood vessel hardness is accurately measured taking into account the dependency of the compressing pressure change $\Delta$P on the blood pressure.

Eighth Embodiment

A blood vessel hardness measurement device according to an eighth embodiment of the present invention will now be described.

In the present embodiment, the blood vessel hardness measurement unit 17 measures the blood vessel hardness based on equation 2, which is shown below, using the above-mentioned corrected value $\Delta$P/BH and the subject's age, weight, and height.

Equation 2

Blood Vessel Hardness = $\{X1 \cdot (\Delta P/BH)\} + (X2 \cdot Age) + (X3 \cdot Weight) + (X4 \cdot Height) + X5$

The equation 2 is a multiple regression equation. In the equation, X1 is a first partial regression coefficient for the corrected value $\Delta$P/BH and the blood vessel hardness, X2 is a second partial regression coefficient for the age and the blood vessel hardness, X3 is a third partial regression coefficient for the weight and the blood vessel hardness, X4 is a fourth partial regression coefficient for the height and the blood vessel hardness, and X5 is a residual error of the multiple regression equation.

The corrected value $\Delta$P/BH, age, weight, height, and blood vessel hardness are obtained for a plurality of subjects to set the first to fourth partial regression coefficients X1 to X4 and the residual error X5. The first to fourth partial regression coefficients X1 to X4 and the residual difference X5 are set in a manner suitable for estimating the blood vessel hardness.

The blood vessel hardness measurement unit 17 measures the blood vessel hardness by substituting, into the equation 2, the corrected value $\Delta$P/BH, which is circulatory information of the subject, and the age, weight, and height, which are the subject's biological information. Such correction functions as a process for weighting the circulatory information and the biological information with the partial regression coefficients to measure the blood vessel hardness.

In addition to advantages (1), (3), (6), (7), and (10), which are mentioned above, the eighth embodiment has the advantage described below.

(11) The blood vessel hardness measurement unit 17 performs correction, or a weighting process that weights the corrected value $\Delta$P/BH, which is the subject's circulatory information, and the age, weight, and height, which is the subject's biological information, using the first to fourth partial regression coefficients, or the weighting coefficients. The blood vessel hardness measurement unit 17 measures the blood vessel hardness using the weighted circulatory information and the weighted biological information. In general, the average value of the blood vessel hardness differs depending on the age, weight, and height of the subject. For example, the average value of the blood vessel hardness has a tendency of increase as the subject's age becomes higher. In this aspect, the device of the present embodiment measures the blood vessel hardness more accurately in accordance with the age, weight, and height of each individual subject even when the same corrected value $\Delta$P/BH is obtained for different subjects.

Ninth Embodiment

A blood vessel hardness measurement device according to a ninth embodiment of the present invention will now be described.

In the present embodiment, the blood vessel hardness measurement unit 17 measures the blood vessel hardness based on equations 3 and 4, which are shown below, using the above-mentioned corrected value $\Delta$P/BH and the subject's age, weight, and height.

Equation 3

Male

Blood Vessel Hardness = {X11·(ΔP/BH}+(X12·Age)+(X13·Weight)+(X14·Height)+X15

Equation 4

Female

Blood Vessel Hardness = {X21·(ΔP/BH}+(X22·Age)+(X23·Weight)+(X24·Height)+X25

The equation 3 is a multiple regression equation. In the equation, X11 is a first partial regression coefficient for the corrected value ΔP/BH and the blood vessel hardness, X12 is a second partial regression coefficient for the age and the blood vessel hardness, X13 is a third partial regression coefficient for the weight and the blood vessel hardness, and X14 is a fourth partial regression coefficient for the height and the blood vessel hardness, and X15 is a residual error of the multiple regression equation.

To set the first to fourth partial regression coefficients X11 to X14 and the residual error X15, the corrected value ΔP/BH, and the age, weight, and height, and the blood vessel hardness are sampled from a number of males. The first to fourth partial regression coefficients X11 to X14 and the residual error X15 are then set in a manner suitable for estimating of the blood vessel hardness of male subjects.

In the same manner, the equation 4 is also a multiple regression equation. In the equation, X21 is a first partial regression coefficient for the corrected value ΔP/BH and the blood vessel hardness, X22 is a second partial regression coefficient for the age and the blood vessel hardness, X23 is a third partial regression coefficient for the weight and the blood vessel hardness, and X24 is a fourth partial regression coefficient for the height and the blood vessel hardness, and X25 is a residual error of the multiple regression equation.

To set the first to fourth partial regression coefficients X21 to X24 and the residual error X25, the corrected value ΔP/BH, and the age, weight, and height, and the blood vessel hardness are sampled from a number of females. The first to fourth partial regression coefficients X21 to X24 and the residual error X25 are then set in a manner suitable for estimating of the blood vessel hardness of female subjects.

In this manner, the blood vessel hardness measurement unit 17 performs the correction for the corrected value ΔP/BH using equation 3 for a male subject and equation 4 for a female subject to measure the blood vessel hardness through the correction.

In addition to advantages (1), (3), (6), (7), (10), and (11), the ninth embodiment has the advantages described below.

(12) The blood vessel hardness measurement unit 17 measures the blood vessel hardness after performing the correction in which the subject's circulatory information and biological information are substituted into equation 3 or equation 4 in accordance with the subject's sex, which is the subject's biological information. In general, the average value of the blood vessel hardness for subjects of the same age, weight, and height differs depending on the sex of the subjects. In this aspect, the device of the present embodiment obtains the blood vessel hardness with further accuracy for different subjects under the same corrected value ΔP/BH in accordance with the subject's sex in addition to the subject's age, weight, and height.

The above embodiments of the present invention may be modified in the following forms.

Tenth Embodiment

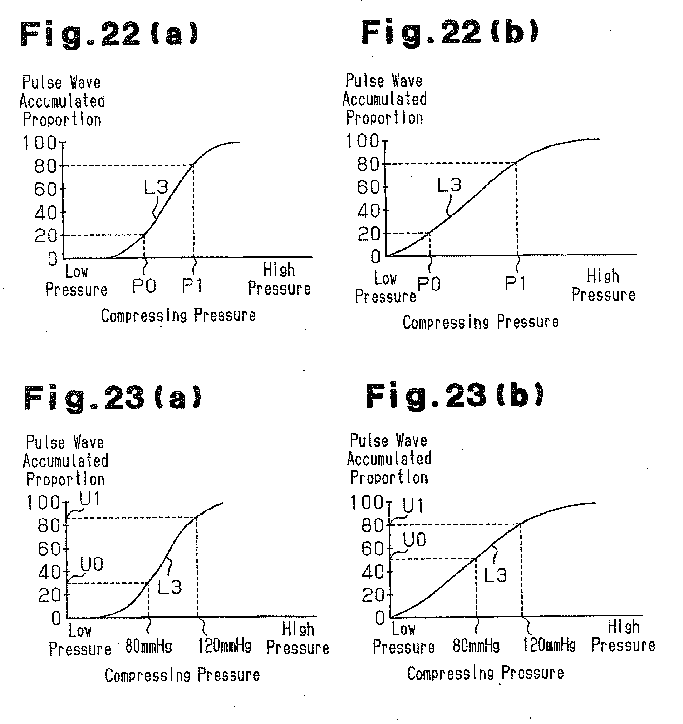

A blood vessel hardness measurement device according to a tenth embodiment of the present invention will now be described with reference to Figs. 22(a) and 22(b).

In the tenth embodiment, the compressing pressure detection unit 13 detects a compressing pressure P0, which corresponds to a pulse wave accumulated proportion of, for example, 20%, and a compressing pressure P1, which corresponds to a pulse wave accumulated proportion of, for example, 80%. The compressing pressure detection unit 13 calculates the ratio of the compressing pressure P0 and the compressing pressure P1 (compressing pressure ratio P1/P0). The blood vessel hardness measurement unit 17 measures the blood vessel hardness based on the compressing pressure ratio P1/P0.

Fig. 22(a) is a graph showing a characteristic line L3 obtained for soft blood vessels. Fig. 22(b) is a graph showing a characteristic line L3 obtained for hardened blood vessels. As apparent from the comparison between the graphs of Figs. 22(a) and 22(b), the compressing pressure ratio P1/P0 of the soft blood vessels is smaller than the compressing pressure ratio P1/P0 of the hard blood vessels. The blood vessel hardness measurement unit 17 includes a map that associates the compressing pressure ratio P1/P0 with the blood vessel hardness. The blood vessel hardness measurement unit 17 uses the map to measure the blood vessel hardness.

The tenth embodiment has the advantage described below.

(13) In the tenth embodiment, the blood vessel hardness is measured using only the compressing pressure ratio P1/P0. This reduces the load on the blood vessel hardness measurement unit 17.

Eleventh Embodiment

A blood vessel hardness measurement device according to an eleventh embodiment of the present invention will now be described with reference to Figs. 23(a) and 23(b).

In the eleventh embodiment, the compressing pressure detection unit 13 measures the blood vessel hardness based on the ratio of a pulse wave accumulated proportion U0, which corresponds to a compressing pressure of, for example, 80 mmHg, and a pulse wave accumulated proportion U1, which corresponds to a compressing pressure of, for example, 120 mmHg (hereafter referred to as the pulse wave accumulated proportion ratio U1/U0).

Fig. 23(a) is a graph showing a characteristic line L3 obtained for soft blood vessels. Fig. 23(b) is a graph showing a characteristic line L3 obtained for hardened blood vessels. As apparent from the comparison between the graphs of Figs. 23(a) and 23(b), the pulse wave accumulated proportion ratio U1/U0 of the soft blood vessels is greater than the pulse wave accumulated proportion ratio U1/U0 of the hard blood vessels. The blood vessel hardness measurement unit 17 includes a map that associates the pulse wave accumulated proportion ratio U1/U0 with the blood vessel hardness. The blood vessel hardness measurement unit 17 uses the map to measure the blood vessel hardness.

The eleventh embodiment has the advantages described below.

(14) In the eleventh embodiment, the blood vessel hardness is measured using only the pulse wave accumulated proportion ratio U1/U0. This reduces the load on the blood vessel hardness measurement unit 17.

It should be apparent to those skilled in the art that the present invention may be embodied in many other specific forms without departing from the spirit or scope of the invention. Particularly, it should be understood that the present invention may be embodied in the following forms.

In the second embodiment, the blood vessel hardness may be measured based on the rate of change of the pulse wave accumulated value relative to a change in the pressure range including the predetermined compressing pressure Ps, which corresponds to the maximum amplitude value of the pulse wave W1 and the maximum change rate of the characteristic line L2. The pressure range around the compressing pressure Ps greatly affects the flexibility of the middle layer 22 of the blood vessel wall 20. Thus, the blood vessel hardness in the pressure range around the compressing pressure Ps is important information for determining the state of the middle layer 22, which forms the most significant part of the blood vessel wall 20. Determining the state of the middle layer 22 enables further accurate measurement of the blood vessel hardness.

In the fourth embodiment, the pulse wave accumulated proportion range may be set to include the predetermined compressing pressure Ps corresponding to the maximum amplitude value of the pulse wave W1 and the maximum change rate of the characteristic line L3. The determination of the state of the middle layer 22 allows for further accurate measurement of the blood vessel hardness.

In the fifth embodiment, the blood vessel hardness measurement unit 17 may measure the blood vessel hardness based on the change $\Delta U$ of the pulse wave accumulated proportion in the pressure range including the predetermined compressing pressure Ps, which corresponds to the maximum amplitude value of the pulse wave W1 and the maximum change rate of the characteristic line L3. The determination of the state of the middle layer 22 allows for further accurate measurement of the blood vessel hardness.

In the fourth embodiment, the compressing pressure detection unit 13 may detect the change $\Delta P$ of the compressing pressure in the pulse wave accumulated proportion range of 10% to 90%. The pulse wave accumulated proportion range is not particularly limited.

In the fifth embodiment, the blood vessel hardness measurement unit 17 may measure the blood vessel hardness based on the change $\Delta U$ of the pulse wave accumulated proportion that changes outside the pressure range including the reference compressing pressure of 40 mmHg. The pressure range of the compressing pressure is not particularly limited.

In the seventh embodiment, the blood vessel hardness measurement unit 17 may measure the blood vessel hardness using a value ($\Delta U/BH$) obtained by dividing the change $\Delta U$ of the pulse wave accumulated proportion in the predetermined pressure range by the subject's systolic pressure BH when the compressing pressure changes in a predetermined range.

In the seventh embodiment, the correction is performed by dividing the compressing pressure change $\Delta P$ by

the subject's systolic pressure BH. However, the compressing pressure change $\Delta P$ may be divided by a value based on the systolic pressure BH, for example, the subject's average blood pressure. This correction enables measurement of the blood vessel hardness taking into account the dependency of the compressing pressure change $\Delta P$ on the blood pressure. The method for such correction is not limited to the above method of dividing the compressing pressure change $\Delta P$ by the systolic pressure BH or by the average blood pressure. For example, the blood vessel hardness may be calculated using a map prepared in advance for calculating the blood vessel hardness using the compressing pressure change $\Delta P$ and the systolic pressure BH (or the average blood pressure) as parameters.

In the seventh embodiment, the systolic pressure BH is used as the biological information for correcting the compressing pressure change $\Delta P$. Alternatively, for example, the age, weight, and height of the subject may be used as the biological information. The body-mass index (BMI), which indicates the ratio of the weight to the height, may also be used as the biological information.

In the eighth embodiment, equation 2 uses the value obtained by dividing the compressing pressure change $\Delta P$ by the subject's systolic pressure BH. However, the compressing pressure change $\Delta P$ may be directly substituted into the equation 2. In this case, the first partial regression coefficient X1 is a different value.

In the eighth embodiment, the value ($\Delta U/BH$) obtained by dividing the change of the pulse wave accumulated proportion in the predetermined compressing pressure range by the subject's systolic pressure BH may be substituted into the equation 2. Alternatively, the pulse wave accumulated proportion change $\Delta U$ may be directly substituted into the equation 2. In these cases, the first partial regression coefficient X1 is a different value.

In the eighth embodiment, the BMI value may be used as the biological information in equation 2. In this case, the equation 2 uses a partial regression coefficient additionally set for the BMI and the blood vessel hardness.

In the ninth embodiment, the partial regression coefficients used in the multiple regression equation for measuring the blood vessel hardness may be changed based on whether the subject has been diagnosed with any disease, such as diabetes.

In the ninth embodiment, reference values may be set for the age, weight, and height of the subject, and the partial regression coefficients may be change based on whether the age, weight, and height of the subject are greater than or equal to the reference values. Alternatively, for example, the age may be categorized into 10 to 19, 20 to 29, 30 to 39, and so on, and the partial regression coefficients may be changed depending on each age category. The same applies to the height and the weight.

In the ninth embodiment, predetermined constants T1 and T2 may be set for males (T1) and females (T2), and the blood vessel hardness may be calculated using the equations given below. In this case, the circulatory information of the subject obtained using the pulse wave accumulated value, or specifically the compressing pressure change $\Delta P$, is corrected based on the subject's sex, which is the subject's biological information.

Equation 5
Male
Blood Vessel Hardness = {X111·($\Delta P/BH$)}+T1

Equation 6
Female
Blood Vessel Hardness = {X211·($\Delta P/BH$)}+T2

In equations 5 and 6, the corrected value $\Delta P/BH$ at the right side may be replaced by the compressing pressure change $\Delta P$.

Although equation 2 uses the subject's age, weight, and height as the subjects biological information, and equations 3 and 4 use the subject's sex in addition to the age, weight, and height as the biological information, the blood vessel hardness may be measured based on the compressing pressure change $\Delta P$ corrected using only one of these items of the biological information.

In the eighth and ninth embodiments of the present invention, the blood vessel hardness is measured using the multiple regression equations. Alternatively, the blood vessel hardness may be measured using any other measurement method that uses the circulatory information and the biological information corrected using the weighting coefficients. For example, the relationship between the biological information including the age, weight, height, and sex and the circulatory information including the compressing pressure change $\Delta P$ may be obtained with a statistical method, such as principle component analysis, and the blood vessel hardness can be measured using the relationship obtained with the statistical method.

Although the pulse wave detection unit 14 generates a pulse wave signal by filtering the compression signal input from the compressing pressure detection unit 13, the pulse wave detection unit 14 may directly detect the pulse wave signal.

In the seventh to ninth embodiments, the change $\Delta P$ of the compressing pressure is divided by the subject's systolic pressure BH to calculate the corrected value $\Delta P/BH$. The values listed below may be used in lieu of the change AP of the compressing pressure.

(A) Compressing pressure ratio P1/P0 or compressing ratio P0/P1

(B) Pulse wave accumulated proportion ratio U1/U0 or pulse wave accumulated proportion ratio U0/U1

(C) Ratio (P1/P0)/(U1/U0) of compressing pressure ratio to pulse wave accumulated proportion ratio

(D) Ratio (P0/P1)/(U0/U1) of compressing pressure ratio to pulse wave accumulated proportion ratio

(E) Ratio (U1/U0)/(P1/P0) of pulse wave accumulated proportion ratio to compressing pressure ratio

(F) Ratio (U0/U1)/(P0/P1) of pulse wave accumulated proportion ratio to compressing pressure ratio

(G) Function log(P1/P0)of compressing pressure ratio, function In(P0/P0) of compressing pressure ratio, or function (P1/P0)2 of compressing pressure ratio

(H) Function log(P0/P1) of compressing pressure ratio, function In(P0/P1) of compressing pressure ratio, or function (P0/P1)2 of compressing pressure ratio

(I) Function log(U1/U0) of pulse wave accumulated proportion ratio, function In(U1/U0) of pulse wave accumulated proportion ratio, or function (U1/U0)2 of pulse wave accumulated proportion ratio

(J) Function log(U0/U1) of pulse wave accumulated proportion ratio, function In(U0/U1) of pulse wave accumulated proportion ratio, or function (U0/U1)2 of pulse wave accumulated proportion ratio

(K) Function log(P1/P0)/(U1/U0) of ratio of compressing pressure ratio to pulse wave accumulated proportion ratio, function In(P1/P0)/(U1/U0) of ratio of compressing pressure ratio to pulse wave accumulated proportion ratio, or function {(P1/P0)/(U1/U0)}2 of ratio of compressing pressure ratio to pulse wave accumulated proportion ratio

(L) Function log(P0/P1)/(U0/U1) of ratio of compressing pressure ratio to pulse wave accumulated proportion ratio, function In(P0/P1)/(U0/U1) of ratio of compressing pressure ratio to pulse wave accumulated proportion ratio, or function {(P0/P1)/(U0/U1)}2 of ratio of compressing pressure ratio to pulse wave accumulated proportion ratio

(M) Function log(U1/U0)/(P1/P0) of ratio of compressing pressure ratio to pulse wave accumulated proportion ratio, function In(U1/U0)/(P1/P0) of ratio of compressing pressure ratio to pulse wave accumulated proportion ratio, or function {(U1/U0)/(P1/P0)}2 of ratio of compressing pressure ratio to pulse wave accumulated proportion ratio

(N) Function log(U0/U1)/(P0/P1) of ratio of compressing pressure ratio to pulse wave accumulated proportion ratio, function In(U0/U1)/(P0/P1) of ratio of compressing pressure ratio to pulse wave accumulated proportion ratio, or function {(U0/U1)/(P0/P1)}2 of ratio of compressing pressure ratio to pulse wave accumulated proportion ratio

In the tenth embodiment and eleventh embodiment, the blood vessel hardness is measured based on the compressing pressure ratio P1/P0 and the pulse wave accumulated proportion ratio U1/U0. However, the blood vessel hardness may be measured based on the values listed below.

(A) Compressing pressure ratio P0/P1

(B) Pulse wave accumulated proportion ratio U0/U1

(C) Ratio (P1/P0)/(U1/U0) of compressing pressure ratio to pulse wave accumulated proportion ratio

(D) Ratio (P0/P1)/(U0/U1) of compressing pressure ratio to pulse wave accumulated proportion ratio

(E) Ratio (U1/U0)/(P1/P0) of pulse wave accumulated proportion ratio to compressing pressure ratio

(F) Ratio (U0/U1)/(P0/P1) of pulse wave accumulated proportion ratio to compressing pressure ratio

(G) Function log(P1/P0)of compressing pressure ratio, function In(P1/P0) of compressing pressure ratio, or function (P1/P0)2 of compressing pressure ratio

(H) Function log(P0/P1) of compressing pressure ratio, function In(P0/P1) of compressing pressure ratio, or function (P0/P1)2 of compressing pressure ratio

(I) Function log(U1/U0) of pulse wave accumulated proportion ratio, function In(U1/U0) of pulse wave accumulated proportion ratio, or function (U1/U0)2 of pulse wave accumulated proportion ratio

(J) Function log(U0/U1) of pulse wave accumulated proportion ratio, function In(U0/U1) of pulse wave accumulated proportion ratio, or function (U0/U1)2 of pulse wave accumulated proportion ratio

(K) Function log(P1/P0)/(U1/U0) of ratio of compressing pressure ratio to pulse wave accumulated proportion ratio, function In(P1/P0)/(U1/U0) of ratio of compressing pressure ratio to pulse wave accumulated proportion ratio, or function {(P1/P0)/(U1/U0)}2 of ratio of compressing pressure ratio to pulse wave accumulated proportion ratio

(L) Function log(P0/P1)/(U0/U1) of ratio of compressing pressure ratio to pulse wave accumulated proportion ratio, function In(P0/P1)/(U1/U0) of ratio of compressing pressure ratio to pulse wave accumulated proportion ratio, or function {(P0/P1)/(U0/U1)}2 of ratio of compressing pressure ratio to pulse wave accumulated proportion ratio

(M) Function log(U1/U0)/(P1/P0) of ratio of compressing pressure ratio to pulse wave accumulated proportion ratio, function In(U1/U)/(P1/P0) of ratio of compressing pressure ratio to pulse wave accumulated proportion ratio, or function {(U1/U0)/(P1/P0)}2 of ratio of compressing pressure ratio to pulse wave accumulated proportion ratio
(N) Function log(U0/U1)/(P0/P1) of ratio of compressing pressure ratio to pulse wave accumulated proportion ratio, function In(U0/U1)/(P/P1) of ratio of compressing pressure ratio to pulse wave accumulated proportion ratio, or function {(U0/U1)/(P0/P1)}2 of ratio of compressing pressure ratio to pulse wave accumulated proportion ratio

In the tenth embodiment, the compressing pressure detection unit 13 detects a compressing pressure P0 which corresponds to a pulse wave accumulated proportion of 20%, and a compressing pressure P1, which corresponds to a pulse wave accumulated proportion of 80%. However, the detection is not particularly limited to two pulse wave accumulated portions.

In the tenth embodiment, the compressing pressure detection unit 13 may detect the compressing pressure ratio P1/P0 based on two compressing pressure values corresponding to two pulse wave accumulated proportions.

In the eleventh embodiment, the blood vessel hardness measurement unit 17 detects the pulse wave accumulated proportion corresponding to the compressing pressure value of 80 mmHg and the pulse wave accumulated proportion corresponding to the compressing pressure value of 120 mmHg. However, the detection is not particularly limited to the two compressing pressures.

In the eleventh embodiment, the blood vessel hardness measurement unit 17 may measure the blood vessel hardness based on a ratio of a pulse wave accumulated value calculated from two pulse wave accumulated values corresponding to the two compressing pressure values of 80 mmHg and 120 mmHg.

In the above embodiment, the blood vessel hardness measurement unit 17 may measure the blood vessel hardness based on a ratio of a pulse wave accumulated value calculated from two pulse wave accumulated values corresponding to two compressing pressure values that are not 80 mmHg and 120 mmHg. The compressing pressure values are not particularly limited.

In the first to sixth embodiments, the tenth embodiment, and the eleventh embodiment, the blood vessel is measured based on a value calculated by the compressing pressure detection unit 13 or blood vessel hardness measurement unit 17 and a map that associates the calculated value with the blood vessel hardness. The value calculated by the compressing pressure detection unit 13 or the blood vessel hardness measurement unit 17 may be inserted into a predetermined function to measure the blood vessel hardness.

In the above embodiments, the pulse wave detection unit 14 may detect the amplitude value of the pulse wave W1 in the period in which the cuff 11 is depressurized gradually after the compressing pressure of the cuff 11 reaches a predetermined pressure higher than the subject's estimated systolic pressure.

In the above embodiments, the pulse wave W1 may be generated from a part of the subject other than the upper arm. For example, the pulse wave W1 may be generated from a wrist of the subject.

## Claims

1. A measurement device comprising:

   a compressing unit (11) that compresses part of a subject's body;
   a compressing pressure detection unit (13) that detects a compressing pressure generated by the compressing unit (11);
   a compressing pressure control unit (12) that changes the compressing pressure;
   a pulse wave detection unit (14) that detects pulse wave information indicating value of a pulse wave generated in the part of the body corresponding to the compressing pressure that is changed by the compressing pressure control unit (12),
   **characterized by**
   a pulse wave accumulated value calculation unit (15) that calculates a pulse wave accumulated value by accumulating plural pieces of the pulse wave information obtained during a period from when the compressing unit (11) starts the compressing to when the compressing unit (11) ends the compressing;
   a memory unit (16) that stores the pulse wave accumulated value in association with the compressing pressure; and
   a measurement unit (17) for measuring the hardness of a blood vessel using the pulse wave accumulated value.

2. The measurement device according to claim 1, wherein the measurement unit (17) measures the hardness of a blood vessel using a characteristic line obtained from a relationship of a plurality of the pulse wave accumulated values and a plurality of the compressing pressures, which are associated with the plurality of pulse wave accumulated values.

3. The measurement device according to claim 2, wherein the measurement unit (17) measures the hardness of a blood vessel based on a rate of change of the pulse wave accumulated value relative to a change of the compressing pressure in the characteristic line.

4. The measurement device according to claim 3, wherein the measurement unit (17) measures the hardness of a blood vessel based on a rate of change of the pulse wave accumulated value in each of a plurality of compressing pressure ranges in the characteristic line.

5. The measurement device according to claim 1, wherein
the measurement unit (17) calculates a plurality of pulse wave accumulated proportions that are proportions of a plurality of pulse wave accumulated values relative to the largest one of the pulse wave accumulated values, and the measurement unit (17) measures the hardness of a blood vessel based on a characteristic curve obtained from a relationship of the plurality of pulse wave accumulated proportions and the corresponding plurality of compressing pressures.

6. The measurement device according to claim 5, wherein the measurement unit (17) measures the hardness of a blood vessel using a change of the compressing pressure in a predetermined range of the pulse wave accumulated proportion in the characteristic line.

7. The measurement device according to claim 5, wherein the measurement unit (17) measures the hardness of a blood vessel based on a change of the pulse wave accumulated proportion in a predetermined range of the compressing pressure in the characteristic line.

8. The measurement device according to any one of claims 2 to 7, wherein the measurement unit (17) measures the hardness of a blood vessel based on a rate of change of the characteristic curve in a range including a compressing pressure at which a rate of change of the characteristic curve is the largest.

9. The measurement device according to any one of claims 1 to 8, wherein the pulse wave accumulated value calculation unit (15) calculates the pulse wave accumulated value by accumulating only plural pieces of pulse wave information from which abnormal pulse wave information detected by the pulse wave detection unit (14) is removed.

10. The measurement device according to any one of claims 1 to 9, wherein
the measurement unit (17) corrects the subject's circulatory information, which is obtained from the pulse wave accumulated value, based on the subject's biological information that differs from the circulatory information, and the measurement unit (17) measures the hardness of a blood vessel using the corrected circulatory information.

11. The measurement device according to claim 10, wherein the measurement unit (17) weights the circulatory information and the biological information when measuring the hardness of a blood vesselusing the corrected circulatory information.

12. The measurement device according to claim 10 or 11, wherein the measurement unit (17) corrects the subject's circulatory information, which includes the subject's sex, using an equation that differs in accordance with the subject's sex when measuring the hardness of a blood vessel using the corrected circulatory information.

13. The measurement device according to any one of claims 5 to 7, wherein the measurement unit (17) measures the hardness of a blood vesselbased on a proportion of two of the compressing pressure values corresponding to two of the pulse wave accumulated proportions in the characteristic line.

14. The measurement device according to any one of claims 5 to 7, wherein the measurement unit (17) measures the hardness of a blood vessel based on a proportion of two of the pulse wave accumulated proportions corresponding to two of the compressing pressure values in the characteristic line.

15. The measurement device according to any one of claims 2 to 12, wherein the measurement unit (17) measures the hardness of a blood vessel based on a proportion of two of the compressing pressure values corresponding to two of the pulse wave accumulated proportions in the characteristic line.

16. The measurement device according to any one of claims 2 to 12, wherein the measurement unit (17) measures the hardness of a blood vessel based on a proportion of two of the pulse wave accumulated proportions corresponding to two of the compressing pressure values in the characteristic line.

**Patentansprüche**

1.  Messvorrichtung mit:

    einer Kompressionseinheit (11), die einen Teil eines Körpers eines Nutzers zusammendrückt;
    einer Kompressionsdruckdetektionseinheit (13), die einen Kompressionsdruck, der durch die Kompressionseinheit (11) erzeugt wird, detektiert;
    einer Kompressionsdrucksteuereinheit (12), die den Kompressionsdruck ändert;
    einer Pulswellendetektionseinheit (14), die Pulswelleninformationen detektiert, die einen Wert einer Pulswelle anzeigen, die in dem Teil des Körpers entsprechend zu dem Kompressionsdruck erzeugt wird, der sich ändert durch die Kompressionsdrucksteuereinheit (12),
    **gekennzeichnet durch**
    eine Berechnungseinheit für einen pulswellenakkumulierten Wert (15), die einen pulswellenakkumulierten Wert berechnet **durch** ein Akkumulieren von mehreren Teilen der Pulswelleninformation, die während einer Periode erhalten werden von einem Moment, wenn die Kompressionseinheit (11) anfängt mit der Kompression, bis zu einem Moment, wenn die Kompressionseinheit (11) aufhört zu komprimieren;
    eine Speichereinheit (16), die den pulswellenakkumulierten Wert speichert, der zu dem Kompressionsdruck gehört; und
    eine Messeinheit (17) zum Messen eines Härtegrades eines Blutgefäßes unter Nutzung des pulswellenakkumulierten Wertes.

2.  Messvorrichtung nach Anspruch 1, wobei die Messeinheit (17) den Härtegrad eines Blutgefäßes misst unter Nutzung einer charakteristischen Linie, die von einer Beziehung von mehreren pulswellenakkumulierten Werten und mehreren der Kornpressionsdrücke erhalten wird, die zugehörig sind zu mehreren pulswellenakkumulierten Werten.

3.  Messvorrichtung nach Anspruch 2, wobei die Messeinheit (17) den Härtegrad eines Blutgefäßes basierend auf einer Änderungsrate des pulswellenakkumulierten Wertes relativ zu einer Änderung des Kompressionsdruckes bei der charakteristischen Linie misst.

4.  Messvorrichtung nach Anspruch 3, wobei die Messeinheit (17) den Härtegrad eines Blutgefäßes basierend auf einer Änderungsrate des pulswellenakkumulierten Wertes in jedem der mehreren Kompressionsdruckbereiche in der charakteristischen Linie misst.

5.  Messvorrichtung nach Anspruch 1, wobei
    die Messeinheit (17) mehrere pulswellenakkumulierte Anteile berechnet, wobei die Anteile Anteile von mehreren pulswellenakkumulierten Werte relativ zu dem größten der pulswellenakkumulierten Werte sind, und
    die Messeinheit (17) den Härtegrad eines Blutgefäßes misst basierend auf einer charakteristischen Kurve, die von einer Beziehung der mehreren pulswellenakkumulierten Anteile und der entsprechenden mehreren Kompressionsdrücke erhalten wird.

6.  Messvorrichtung nach Anspruch 5, wobei die Messeinheit (17) den Härtegrad des Blutgefäßes misst unter Nutzung einer Änderung eines Kompressionsdruckes in einem vorbestimmten Bereich des pulswellenakkumulierten Anteils in der charakteristischen Linie.

7.  Messvorrichtung nach Anspruch 5, wobei die Messeinheit (17) den Härtegrad eines Blutgefäßes misst basierend auf einer Änderung eines pulswellenakkumulierten Anteiles in einem vorbestimmten Bereich des Kompressionsdruckes in der charakteristischen Linie.

8.  Messvorrichtung nach einem der Ansprüche 2 bis 7, wobei die Messeinheit (17) den Härtegrad eines Blutgefäßes misst basierend auf einer Änderungsrate der charakteristischen Kurve in einem Bereich umfassend einen Kompressionsdruck, bei welchem eine Änderungsrate der charakteristischen Kurve am größten ist.

9.  Messvorrichtung nach einem der Ansprüche 1 bis 8, wobei die Berechnungseinheit für den pulswellenakkumulierten Wert (15) den pulswellenakkumulierten Wert berechnet durch eine Akkumulation von lediglich mehreren Anteilen einer Pulswelleninformation, von welcher abnorme Pulswelleninformation detektiert wurde durch die Pulswellendetektionseinheiten (14) entfernt wurde.

10. Messvorrichtung nach einem der Ansprüche 1 bis 9, wobei die Messeinheit (17) Kreislaufinformation eines Nutzers korrigiert, die erhalten wird von dem pulswellenakkumulierten Wert basierend auf biologischen Informationen des Nutzers, die sich von den Kreislaufinformationen unterscheiden, und die Messeinheit (17) den Härtegrad eines Blutgefäßes unter Nutzung der korrigierten Kreislaufinformation misst.

11. Messvorrichtung nach Anspruch 10, wobei die Messeinheit (17) die Kreislaufinformation und die biologische Information gewichtet, wenn der Härtegrad eines Blutgefäßes unter Nutzung der korrigierten Kreislaufinformation gemessen wird.

12. Messvorrichtung nach Anspruch 10 oder 11, wobei die Messeinheit (17) die Kreislaufinformation eines Nutzers korrigiert, die das Geschlecht des Nutzers umfasst, unter Nutzung einer Gleichung, die entsprechend zu dem Geschlecht des Nutzers sich ändert, wenn der Härtegrad des Blutgefäßes unter Nutzung der korrigierten Kreislaufinformation gemessen wird.

13. Messvorrichtung nach einem der Ansprüche 5 bis 7, wobei die Messeinheit (17) den Härtegrad eines Blutgefäßes basierend auf einen Anteil von zwei der Kompressionsdruckwerte entsprechend zu zwei der pulswellenakkumulierten Anteile in der charakteristischen Kurve misst.

14. Messvorrichtung nach einem der Ansprüche 5 bis 7, wobei die Messeinheit (17) den Härtegrad eines Blutgefäßes basierend auf einem Anteil von zwei der pulswellenakkumulierten Anteile entsprechend zu zwei der Kompressionsdruckwerte in der charakteristischen Linie misst.

15. Messvorrichtung nach einem der Ansprüche 2 bis 12, wobei die Messeinheit (17) den Härtegrad eines Blutgefäßes basierend auf einem Anteil von zwei der Kompressionsdruckwerte entsprechend zu zwei der pulswellenakkumulierten Anteile in der charakteristischen Kurve misst.

16. Messvorrichtung nach einem der Ansprüche 2 bis 12, wobei die Messeinheit (17) den Härtegrad eines Blutgefäßes basierend auf einem Anteil von zwei der pulswellenakkumulierten Anteile entsprechend zu zwei der Kompressionsdruckwerte in der charakteristischen Linie misst.

**Revendications**

1. Dispositif de mesure, comprenant :

une unité de compression (11) qui comprime une partie du corps d'un sujet ;
une unité de détection de pression de compression (13) qui détecte une pression de compression générée par l'unité de compression (11) ;
une unité de commande de pression de compression (12) qui change la pression de compression ;
une unité de détection d'onde de pouls (14) qui détecte des informations d'onde de pouls indiquant la valeur d'une onde de pouls générée dans la partie du corps correspondant à la pression de compression qui est changée par l'unité de commande de pression de compression (12),
**caractérisé par**
une unité de calcul de valeur cumulée d'onde de pouls (15) qui calcule une valeur cumulée d'onde de pouls en cumulant plusieurs éléments d'information d'onde de pouls obtenus au cours d'une période partant du moment où l'unité de compression (11) commence la compression jusqu'au moment où l'unité de compression (11) achève la compression ;
une unité de mémoire (16) qui stocke la valeur cumulée d'onde de pouls en association avec la pression de compression ; et
une unité de mesure (17) pour mesurer la rigidité d'un vaisseau sanguin au moyen de la valeur cumulée d'onde de pouls.

2. Dispositif de mesure selon la revendication 1, dans lequel l'unité de mesure (17) mesure la rigidité d'un vaisseau sanguin au moyen d'une ligne caractéristique obtenue à partir d'une relation d'une pluralité de valeurs cumulées d'onde de pouls et d'une pluralité de pressions de compression, qui sont associées à la pluralité de valeurs cumulées d'onde de pouls.

**3.** Dispositif de mesure selon la revendication 2, dans lequel l'unité de mesure (17) mesure la rigidité d'un vaisseau sanguin sur la base d'une vitesse de changement de la valeur cumulée d'onde de pouls par rapport à un changement de la pression de compression de la ligne caractéristique.

**4.** Dispositif de mesure selon la revendication 3, dans lequel l'unité de mesure (17) mesure la rigidité d'un vaisseau sanguin sur la base d'une vitesse de changement de la valeur cumulée d'onde de pouls dans chacune d'une pluralité de plages de pression de compression de la ligne caractéristique.

**5.** Dispositif de mesure selon la revendication 1, dans lequel
l'unité de mesure (17) calcule une pluralité de proportions cumulées d'onde de pouls qui sont les proportions d'une pluralité de valeurs cumulées d'onde de pouls par rapport à la valeur cumulée la plus importante d'entre les valeurs cumulées d'onde de pouls, et
l'unité de mesure (17) mesure la rigidité d'un vaisseau sanguin sur la base d'une courbe caractéristique obtenue à partir d'une relation de la pluralité de proportions cumulées d'onde de pouls et de la pluralité de pressions de compression correspondantes.

**6.** Dispositif de mesure selon la revendication 5, dans lequel l'unité de mesure (17) mesure la rigidité d'un vaisseau sanguin au moyen d'un changement de la pression de compression dans une plage prédéterminée de la proportion cumulée d'onde de pouls de la ligne caractéristique.

**7.** Dispositif de mesure selon la revendication 5, dans lequel l'unité de mesure (17) mesure la rigidité d'un vaisseau sanguin sur la base d'un changement de la proportion cumulée d'onde de pouls dans une plage prédéterminée de la pression de compression de la ligne caractéristique.

**8.** Dispositif de mesure selon l'une quelconque des revendications 2 à 7, dans lequel l'unité de mesure (17) mesure la rigidité d'un vaisseau sanguin sur la base d'une vitesse de changement de la courbe caractéristique dans une plage incluant une pression de compression à laquelle une vitesse de changement de la courbe caractéristique est la plus importante.

**9.** Dispositif de mesure selon l'une quelconque des revendications 1 à 8, dans lequel l'unité de calcul de valeurs cumulées d'onde de pouls (15) calcule la valeur cumulée d'onde de pouls en cumulant seulement plusieurs éléments d'information d'onde de pouls desquels les informations d'onde de pouls anormales détectées par l'unité de détection d'onde de pouls (14) sont supprimées.

**10.** Dispositif de mesure selon l'une quelconque des revendications 1 à 9, dans lequel
l'unité de mesure (17) corrige les informations circulatoires du sujet, qui sont obtenues à partir de la valeur cumulée d'onde de pouls, sur la base des informations biologiques du sujet qui diffèrent des informations circulatoires, et
l'unité de mesure (17) mesure la rigidité d'un vaisseau sanguin au moyen des informations circulatoires corrigées.

**11.** Dispositif de mesure selon la revendication 10, dans lequel l'unité de mesure (17) pondère les informations circulatoires et les informations biologiques lors de la mesure de la rigidité d'un vaisseau sanguin, au moyen des informations circulatoires corrigées.

**12.** Dispositif de mesure selon la revendication 10 ou 11, dans lequel l'unité de mesure (17) corrige les informations circulatoires du sujet, qui incluent le sexe du sujet, au moyen d'une équation qui diffère en fonction du sexe du sujet lors de la mesure de la rigidité d'un vaisseau sanguin au moyen des informations circulatoires corrigées.

**13.** Dispositif de mesure selon l'une quelconque des revendications 5 à 7, dans lequel l'unité de mesure (17) mesure la rigidité d'un vaisseau sanguin sur la base d'une proportion de deux des valeurs de pression de compression correspondant à deux des proportions cumulées d'onde de pouls de la ligne caractéristique.

**14.** Dispositif de mesure selon l'une quelconque des revendications 5 à 7, dans lequel l'unité de mesure (17) mesure la rigidité d'un vaisseau sanguin sur la base d'une proportion de deux des proportions cumulées d'onde de pouls correspondant à deux des valeurs de pression de compression de la ligne caractéristique.

**15.** Dispositif de mesure selon l'une quelconque des revendications 2 à 12, dans lequel l'unité de mesure (17) mesure la rigidité d'un vaisseau sanguin sur la base d'une proportion de deux des valeurs de pression de compression correspondant à deux des proportions cumulées d'onde de pouls de la ligne caractéristique.

16. Dispositif de mesure selon l'une quelconque des revendications 2 à 12, dans lequel l'unité de mesure (17) mesure la rigidité d'un vaisseau sanguin sur la base d'une proportion de deux des proportions cumulées d'onde de pouls correspondant à deux des valeurs de pression de compression de la ligne caractéristique.

**Fig.1**

# Fig.2

Compressing Pressure

W1

Elapsed Time

# Fig.3

Pulse Wave Amplitude Value

L1

X3

X2 X1

Low Pressure

P1

P2

P3

Ps

High Pressure

Compressing Pressure

# Fig.4

Pulse Wave Accumulated Value

$X1+X2+X3$

$X1+X2$

$X1$

P1   P2   P3

Compressing Pressure

## Fig.5

## Fig.6

## Fig.7

# Fig.8(a)

Pulse Wave Accumulated Value

L2

60 100 140
Compressing Pressure (mmHg)

# Fig.8(b)

| Pressure | Blood Vessel Hardness |
|---|---|
| 60 | Hard |
| 100 | Soft |
| 140 | Hard |

# Fig.9(a)

Pulse Wave Accumulated Value

L2

60 100 140
Compressing Pressure (mmHg)

# Fig.9(b)

| Pressure | Blood Vessel Hardness |
|---|---|
| 60 | Hard |
| 100 | Hard |
| 140 | Hard |

# Fig.10(a)

Pulse Wave Accumulated Value

L2

60 100 140
Compressing Pressure (mmHg)

# Fig.10(b)

| Pressure | Blood Vessel Hardness |
|---|---|
| 60 | Soft |
| 100 | Soft |
| 140 | Soft |

# Fig.11(a)

Pulse Wave Accumulated Value

L2

60 100 140
Compressing Pressure (mmHg)

# Fig.11(b)

| Pressure | Blood Vessel Hardness |
|---|---|
| 60 | Soft |
| 100 | Hard |
| 140 | Soft |

# Fig.12(a)

Pulse Wave
Accumulated Value

L2

60 100 140
Compressing Pressure (mmHg)

# Fig.12(b)

| Pressure | Blood Vessel Hardness |
|---|---|
| 60 | Soft |
| 100 | Soft |
| 140 | Hard |

# Fig.13(a)

Pulse Wave
Accumulated Value

L2

60 100 140
Compressing Pressure (mmHg)

# Fig.13(b)

| Pressure | Blood Vessel Hardness |
|---|---|
| 60 | Hard |
| 100 | Hard |
| 140 | Soft |

# Fig.14(a)

Pulse Wave
Accumulated Value

L2

60 100 140
Compressing Pressure (mmHg)

# Fig.14(b)

| Pressure | Blood Vessel Hardness |
|---|---|
| 60 | Hard |
| 100 | Soft |
| 140 | Soft |

# Fig.15(a)

Pulse Wave
Accumulated Value

L2

60 100 140
Compressing Pressure (mmHg)

# Fig.15(b)

| Pressure | Blood Vessel Hardness |
|---|---|
| 60 | Soft |
| 100 | Hard |
| 140 | Hard |

# Fig.16

Pulse Wave Accumulated Proportion

100
80
60
40
20
0

L3

ΔU

ΔP

Low Pressure

Ps

High Pressure

Compressing Pressure

# Fig.17(a)

Blood Vessel Volume

Pulse Wave

0

Pulse Pressure

Pressure Difference Between Inner and Outer Sides of Blood Vessel

# Fig.17(b)

Pulse Wave Height

L4

Pressure Difference Between Inner and Outer Sides of Blood Vessel

# Fig.18(a)

Blood Vessel Volume

Pulse Wave

0

Pulse Pressure

Pressure Difference Between Inner and Outer Sides of Blood Vessel

# Fig.18(b)

Pulse Wave Height

L5

Pressure Difference Between Inner and Outer Sides of Blood Vessel

# Fig.19(a)

Pulse Wave
Accumulated
Proportion

100
80
60
40
20
0

L3

Low
Pressure

ΔP

High
Pressure

Compressing Pressure

# Fig.19(b)

Pulse Wave
Accumulated
Proportion

100
80
60
40
20
0

L3

Low
Pressure

ΔP

High
Pressure

Compressing Pressure

# Fig.20(a)

Pulse Wave
Accumulated
Proportion

100
80
60
40
20
0

ΔU

L3

Low
Pressure

40mmHg

High
Pressure

Compressing Pressure

# Fig.20(b)

Pulse Wave
Accumulated
Proportion

100
80
60
40
20
0

ΔU

L3

Low
Pressure

40mmHg

High
Pressure

Compressing Pressure

# Fig.21

Rmax

L10

Pulse Wave
Amplitude Value

R1

Low Pressure

Ps

High Pressure

Compressing Pressure

# Fig.22(a)

Pulse Wave
Accumulated
Proportion

Compressing Pressure

# Fig.22(b)

Pulse Wave
Accumulated
Proportion

Compressing Pressure

# Fig.23(a)

Pulse Wave
Accumulated
Proportion

Compressing Pressure

# Fig.23(b)

Pulse Wave
Accumulated
Proportion

Compressing Pressure

28

**EP 2 465 422 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2005278708 A
- JP 2007044364 A